# FASCICULE DE BREVET EUROPEEN

(11) **EP 2 846 873 B1**
(45) Date de publication et mention de la délivrance du brevet: **12.07.2017**
(21) Numéro de dépôt: 13723077.7
(22) Date de dépôt: 10.05.2013
(51) Int. Cl.: A61P 17/00, A61K 9/107, A61K 9/00, A61K 33/04

(54) **THIOSULFATE DE SODIUM POUR LE TRAITEMENT DES CALCIFICATIONS ECTOPIQUES**
NATRIUMTHIOSULFAT FÜR DIE BEHANDLUNG VON EKTOPISCHEN KALZIFIZIERUNGEN
SODIUM THIOSULFATE FOR THE TREATMENT OF ECTOPIC CALCIFICATIONS

(30) Priorité: 10.05.2012 EP 12305517
(43) Date de publication de la demande: 18.03.2015
(73) Titulaire: Institut National de la Santé et de la Recherche Médicale, 75013 Paris (FR); Universite de Limoges, 87032 Limoges Cedex 01 (FR); Chu De Limoges, 87042 Limoges (FR); Université Paris-Sud, 91405 Orsay Cedex (FR)
(72) Inventeur: RATSIMBAZAFY, Voa, F-87000 Limoges (FR); JOST, Jeremy, F-87000 Limoges (FR); GUIGONIS, Vincent, F-87025 Limoges (FR); CAUDRON, Eric, F-75015 Paris (FR)
(74) Mandataire: Lavoix
(86) Numéro de dépôt international: PCT/EP2013/059744
(87) Numéro de publication internationale: WO 2013/167741

(56) Documents cités:
- WO-A1-2009/103069
- WO-A1-2011/005841
- FR-A- 1 005 574
- US-A1- 2002 164 381

## Description

La présente invention concerne des compositions pharmaceutiques comprenant du thiosulfate de sodium dispersé dans une émulsion hydrophile-dans-lipophile, et leur utilisation pour une administration topique pour le traitement d'une calcification ectopique et/ou de ses conséquences chez un individu, le thiosulfate de sodium étant sous la forme d'une composition pharmaceutique comprenant en outre une émulsion hydrophile-dans-lipophile. L'invention concerne également un procédé de préparation de ces compositions pharmaceutiques. L'invention est définie dans les revendications annexées. Toute divulgation allant au-delà de la portée desdites revendications est uniquement destinée à des fins d'illustration.

### CONTEXTE DE L'INVENTION

Les calcifications cutanées et sous-cutanées (appelées d'une façon générale calcifications ectopiques) compliquent de nombreuses maladies. Elles peuvent être classées en calcifications dystrophiques, métastatiques, idiopathiques, iatrogéniques ou en calciphylaxie.

Les calcifications dystrophiques résultent d'anomalies tissulaires locales et se développent malgré des taux plasmatiques de calcium et de phosphore normaux. Les principales maladies pouvant se compliquer de ces calcifications sont : les connectivites (sclérodermie, CREST syndrome, dermatomyosite juvénile, lupus), les infections cutanées et sous-cutanées (panniculites), les tumeurs cutanées (notamment les pilomatricomes), certaines pathologies congénitales (maladie d'Elher-Danlos, syndrome de Werner, pseudo xanthome élastique).

Les calcifications métastatiques sont par contre la résultante d'un trouble du métabolisme phosphocalcique (hypercalcémie et/ou hyperphosphatémie). Toutes les maladies à l'origine de ces troubles peuvent donc favoriser l'apparition de calcifications.

Les calcifications idiopathiques surviennent sans lésions tissulaires ni trouble du métabolisme phosphocalcique. Les principales pathologies connues dans ce groupe sont la calcinose tumorale, les calcifications scrotales ainsi que les nodules sous épidermiques calcifiés.

Les calcifications iatrogènes peuvent survenir suite à l'injection de calcium ou d'acide para-aminosalycylique. Elles ont également été décrites suite à l'utilisation d'électrodes saturées en chlorure de calcium.

La calciphylaxie correspond à la calcification de vaisseaux de petits calibres et du tissu adipeux sous-cutané. La plupart du temps secondaire à une insuffisance rénale chronique, et souvent associée à des anomalies du métabolisme phosphocalcique, elle reste cependant une entité à part compte tenu de sa physiopathologie spécifique et de ses modalités évolutives propres.

Au-delà du caractère anormal et inesthétique de leur présence, ces calcifications peuvent se compliquer sur le plan fonctionnel (limitation des amplitudes articulaires), sur le plan douloureux (caractère très douloureux des certaines calcifications, notamment dans la calciphylaxie) ou sur le plan trophique (ischémie et nécrose des tissus cutanés et sous cutanés), pouvant - par ce biais - aboutir à des complications infectieuses surajoutées.

Même si de nombreux traitements ont été essayés et rapportés (bisphosphonates, inhibiteurs calciques, probénécide) pour ces calcifications cutanées et sous cutanées, il n'existe à ce jour aucun traitement curatif pour lequel une efficacité ait été démontrée avec un niveau de preuve suffisant.

Le sodium thiosulfate est approuvé aux Etats-Unis en tant qu'antidote d'une intoxication aux cyanures, et en prophylaxie de la néphrotoxicité du cisplatine. Plusieurs publications ont suggéré un effet potentiel du thiosulfate de sodium pour le traitement de calcifications ectopiques, en particulier de la calciphylaxie. Dans le cadre de son autorisation de mise sur le marché comme antidote des cyanures et dans la plupart des cas publiés, le thiosulfate de sodium a été administré préférentiellement par voie intraveineuse. Certains articles rapportent son utilisation par voie orale. L'éventuelle utilisation du thiosulfate de sodium par voie générale pour le traitement de calcifications causées par exemple par des lithiases urinaires, des néphrocalcinoses, des calcinoses tumorales, des calciphylaxies, ou des dermopathies néphrogéniques fibrosantes, suscite un intérêt grandissant. Mais des inquiétudes subsistent concernant d'éventuels effets secondaires systémiques avec ces voies générales (troubles digestifs, acidose métabolique, conséquences osseuses générales...).

Il existe également une lotion concentrée à 25% de thiosulfate de sodium pour application cutanée, associé à l'acide acétylsalicylique, connue sous le nom de Versiclear®, autorisée aux Etats-Unis et indiquée dans le traitement du pytiriasis versicolor. Dans ses excipients, on note la présence d'alcool isopropylique et de propylène glycol.

L'utilisation topique du thiosulfate de sodium pourrait être avantageuse, en particulier pour traiter des calcifications ectopiques.

Deux articles rapportent les effets d'une administration de thiosulfate de sodium par voie cutanée. Le premier concerne une femme de 41 ans, atteinte d'un lupus érythémateux disséminé et présentant un livedo reticularis avec ulcères au niveau des tibias, les lésions étant compliquées d'une infection, et d'une calcification dystrophique (Wolf et al., Arch Dermatol 2008;144:1560-2). Un traitement cutané par thiosulfate de sodium a été mis en oeuvre deux fois par semaine, les soins étaient réalisés au cabinet avec application de compresses à 10% de thiosulfate de sodium avant pansement. Ce traitement était complété par l'application d'un topique corticoïde autour de la plaie. De plus, la patiente appliquait quotidiennement des compresses d'acide acétique avant pansement. Son traitement était complété par une antibiothérapie orale adaptée. Après six mois, les calcifications superficielles se sont dissoutes, les douleurs s'étaient parallèlement progressivement amendées et l'antibiothérapie a pu être allégée. Un trimestre plus tard, une ré-épithélialisation quasi complète s'était opérée au niveau des plaies et l'antibiothérapie a pu être arrêtée.

Le deuxième article rapporte deux autres cas traités par voie cutanée. Le premier concerne une femme de 74 ans, atteinte d'ostéo-arthrite, de sclérose en plaques et d'un pseudo-hypoparathyroidisme, et présentant un ulcère à la jambe avec des dépôts de calcium cutanés et une fibrose (Bair et al., J Drugs Dermatol. 2011;10:1042-4). La patiente a reçu des applications topiques de thiosulfate de sodium à 25% dans de l'oxyde de zinc deux fois par jour au niveau de la plaie et sur la peau autour. Ce traitement était complété par l'utilisation de bandes élastiques, favorisant la compression et l'élévation de la jambe. L'état de la plaie s'est fortement amélioré après cinq semaines, avec une bonne ré-épithélialisation, et a complètement guéri après 15 semaines de thérapie continue. Le second cas concerne un homme de 81 ans, présentant un taux de calcium sérique élevé, une légère insuffisance rénale et un ulcère calcifié évoquant une calcification dystrophique à la jambe. Ce patient a reçu le même traitement que la patiente précédente et a été complètement guéri après 12 semaines de thérapie.

Ces deux publications montrent que l'administration cutanée de thiosulfate de sodium permet de traiter des ulcères de la jambe présentant des calcifications dystrophiques cutanées. En revanche, ces articles ne donnent pas de détails précis concernant la formulation du thiosulfate de sodium.

Il existe deux hypothèses principales pour expliquer l'efficacité du thiosulfate. La première consisterait en une chélation du calcium fixé sur les vaisseaux, dont le produit, le thiosulfate de calcium hautement soluble est ensuite éliminé par voie rénale. Le deuxième mécanisme impliquerait la propriété antioxydante du thiosulfate de sodium (lequel possède deux électrons non appariés), qui contribue à la restauration de la fonction endothéliale et à la production de l'enzyme eNOS.

D'autres hypothèses sont également envisageables. La molécule de thiosulfate de sodium possède en effet deux électrons non appariés, disponibles pour réagir avec les espèces réactives à l'oxygène générées lors de la dysfontion endothéliale qui accompagne la calciphylaxie. Une production d'un antioxydant physiologique, le glutathion (GSH) est observée au cours de cette réaction. Le thiosulfate de sodium pourrait également être à l'origine d'une émission de H₂S, gaz modulateur neurovasculaire, à partir de diverses réactions mettant en jeu des composés thiols avec des réactions de trans-sulfurations enzymatiques à partir du substrat endogène que représente la L-cystéine. Cette molécule, H₂S, est reconnue comme étant dotée de propriétés vasodilatatrice, antalgique et anti-inflammatoire qui peuvent expliquer le soulagement des douleurs observé dans tous les cas rapportés. L'amélioration de la dysfonction endothéliale pourrait également expliquer l'intense et rapide soulagement des douleurs neuropathiques rapporté.

Les propriétés chélatrices du thiosulfate de sodium vis-à-vis du calcium seraient à l'origine de son action sur les calcifications sous-cutanées se manifestant sous la forme de nodules ou plaques douloureux, action sur un long terme, nécessitant des mois de traitement, mais dont la réalité est objectivée par l'examen physique (palpation) et l'imagerie.

Les inventeurs ont mis au point une composition pharmaceutique comprenant du thiosulfate de sodium dispersé dans une émulsion hydrophile-dans-lipophile telle que décrite dans la revendication 1. Ils ont également administré cette préparation par voie cutanée à un garçon âgé de 12 ans présentant une calcification sous-cutanée de grande taille au niveau de la face postérieure de son coude gauche, laquelle limitait la mobilisation du coude. Le patient a appliqué localement chaque soir environ 1 à 1,5 gramme de la composition pharmaceutique contenant une teneur en thiosulfate de sodium de 10% (en poids). Le patient n'a reçu aucun autre type de traitement durant son traitement par le thiosulfate de sodium. Après six mois de traitement, l'examen médical a montré une amélioration drastique, aucune lésion sous-cutanée n'étant visible et la mobilisation du coude étant redevenue normale. De plus, aucun effet secondaire, ni général, ni local, n'a été observé. Ces données montrent que le thiosulfate de sodium peut être utilisé pour une administration topique pour permettre un traitement efficace et sûr des calcifications des tissus mous.

Par conséquent, l'invention porte sur l'utilisation du thiosulfate de sodium pour une administration topique pour le traitement d'une calcification ectopique et/ou de ses conséquences chez un individu recevant ou pas d'autre médication pharmacologique ou mécanique préventive ou thérapeutique des calcifications. L'invention concerne également la composition pharmaceutique comprenant du thiosulfate de sodium dispersé dans une émulsion hydrophile-dans-lipophile mise au point par les inventeurs, ainsi que le procédé de préparation de cette composition pharmaceutique.

WO2009/103069 A1, US2002/164381 A1, FR1005574 A décrivent des compositions comprenant du thiosulfate de sodium.

WO2011/005841 A1 a trait à des compositions pharmaceutiques comprenant du thiosulfate de sodium comme principe actif.

### DESCRIPTION DE L'INVENTION

### Utilisation du thiosulfate de sodium pour le traitement de calcifications ectopiques et/ou de leurs conséquences par administration topique

L'invention concerne tout d'abord le thiosulfate de sodium pour son utilisation pour une administration topique pour le traitement d'une calcification ectopique et/ou de ses conséquences chez un individu, le thiosulfate de sodium étant sous la forme d'une composition pharmaceutique comprenant en outre une émulsion hydrophile-dans-lipophile telle que décrite dans la revendication 1. Le terme « thiosulfate de sodium » désigne tout composé de formule anhydre Na₂S₂O₃ ou tout composé communément connu sous l'une des appellations suivantes : sodium thiosulfate, hyposulfate de sodium, sel di-sodique de l'acide thiosulfurique, sodium hyposulfate, natrii thiosulfas, sodium thiosulphate, natrium thiosulfuricum, natriumthiosulfat.

Le thiosulfate de sodium commercialisé est souvent hydraté, généralement pentahydraté Na₂S₂O₃, 5H₂O (CAS number 10102-17-7 : IPCS, 1993). De formule brute H₁₀Na₂O₈S₂, sa masse moléculaire est de 248,2. Le thiosulfate de sodium pentahydraté est un produit solide qui se présente sous forme granulaire ou de cristaux, incolores ou blancs, et inodores. Légèrement hygroscopique, il est devient déliquescent en atmosphère humide. Le thiosulfate de sodium pentahydraté fond à 45°C, se dissout dans son eau de cristallisation vers 49°C et est très soluble dans l'eau, 780g/l à 20°C. A 100°C, il perd ses molécules d'eau, se décompose au-delà en dégageant des oxydes de soufre et de l'oxyde de sodium.

Le sodium thiosulfate se décompose au contact des acides en émettant du dioxyde de soufre, toxique. Il entre en réaction avec les iodures, les sels de mercure, d'argent, de plomb et d'autres métaux lourds ainsi que les agents oxydants. D'ailleurs il peut exploser au contact des oxydants forts (chlorates, nitrates ou permanganates).

Les solutions aqueuses se décomposent lentement, selon une réaction accélérée par les acides, en Na₂S₂O₃ → Na₂SO₃ + S et, accélérée en présence d'oxygène, en Na₂S₂O₃ + H₂O → Na₂SO₄ + H₂S. Si le pH calculé d'une solution aqueuse saturée est de 8,1, les solutions concentrées à 10% ont un pH avoisinant la neutralité, entre 6,0 et 8,4. Le pKa1 se situe entre 1,46 et 1,74. Une solution injectable à 0,15g/mL additionnée de sodium phosphate dodécahydrate (Na₂HPO₄.12H₂O) à 1,21% a quant à elle, un pH entre 8,2 et 8,8. Conditionnée en ampoule, elle est alors stable 3 ans. Ces solutions se décomposent encore plus rapidement à la chaleur. Le thiosulfate de sodium pentahydraté doit être conservé en évitant le contact avec l'air, l'exposition à la lumière et dans un environnement frais, à l'abri des acides et des substances oxydantes.

L' « individu » à traiter est en particulier un mammifère, de préférence un humain. L'individu peut par exemple être un adulte ou un enfant. L'individu peut recevoir ou non une autre médication pharmacologique ou mécanique, préventive ou thérapeutique des calcifications, en plus de la composition pharmaceutique selon l'invention. Selon un mode de réalisation particulier, l'individu ne reçoit pas d'autre médication pharmacologique ou mécanique, préventive ou thérapeutique des calcifications, en plus de la composition pharmaceutique selon l'invention.

Par « administration topique », on entend toute administration par voie locale, par exemple sur la peau, un orifice, ou une muqueuse. L'administration topique, telle qu'utilisée ici, comprend la voie cutanée, auriculaire, nasale, vaginale, urétrale, et rectale.

Le thiosulfate de sodium est formulé dans une crème, forme pharmaceutique qui se prête particulièrement bien à une administration topique pour un effet local.

Les inventeurs ont rapporté que l'administration par voie topique d'une composition pharmaceutique comprenant du thiosulfate de sodium et une émulsion hydrophile-dans-lipophile telle que décrite dans la revendication 1, sans association à d'autres traitements, a permis de réduire de façon drastique une calcification sous-cutanée de grande taille au niveau du coude d'un garçon de douze ans atteint d'un syndrome de calcinose tumorale. Par conséquent, une composition pharmaceutique comprenant du thiosulfate de sodium et une émulsion hydrophile-dans-lipophile peut être utilisée avantageusement par voie topique pour traiter un individu présentant une ou des calcification(s) ectopique(s). De plus, la composition pharmaceutique selon l'invention peut être utilisée préférentiellement seule, c'est-à-dire non associée à d'autres traitements thérapeutiques.

L'expression « calcification ectopique » désigne tout dépôt pathologique de sels de calcium ou toute croissance osseuse dans les tissus, en particulier dans un tissu mou ou toute autre localisation accessible à un traitement par voie topique. Par « tissu mou », on entend un tissu, autre que le tissu osseux, qui relie, soutient, ou entoure d'autres structures et organes du corps. Les tissus mous comprennent les tendons, les ligaments, les fascias, la peau, les tissus fibreux, les tissus adipeux, des membranes synoviales, les muscles, les nerfs et les vaisseaux sanguins.

La peau a une structure constituée de trois couches : l'épiderme, le derme et l'hypoderme ou tissu sous-cutané. Le tissu sous-cutané contient le tissu adipeux et conjonctif qui abrite les gros vaisseaux sanguins et les nerfs. La plupart des calcifications vasculaires et ectopiques sont situées dans les tissus cutané et sous-cutané. De plus, ces tissus sont particulièrement faciles d'accès pour permettre une administration topique du thiosulfate de sodium. Par conséquent, la calcification à traiter est préférentiellement une calcification cutanée ou sous-cutanée.

Le thiosulfate de sodium administré par voie topique peut permettre le traitement de n'importe quelle calcification ectopique, quelle qu'en soit l'origine. En particulier, le thiosulfate de sodium peut être utilisé pour traiter des calcifications métastatiques, dystrophiques, iatrogènes, ou idiopathiques, des calcifications associées à de la calciphylaxie, ou des ossifications ectopiques sous-cutanées.

Le thiosulfate de sodium peut être utilisé préférentiellement pour traiter des calcifications associées à une maladie ou condition pathologique sélectionnée dans le groupe constitué de l'hyperparathyroidie primaire, l'intoxication à la vitamine D, le syndrome des buveurs de lait, l'hypercalcémie, l'hyperparathyroidie secondaire, l'insuffisance rénale, les hyperphosphatémies, en particulier génétiques, la sclérodermie, la dermatomyosite, en particulier la forme juvénile, les connectivités mixtes, le lupus, le syndrome CREST, le syndrome d'Elhers-Danlos, le pseudo xanthome élastique, le syndrome de Werner, les porphyries cutanées tardives, la pseudo hypoparathyroidie, la pseudo-pseudo hypoparathyroidie, l'insuffisance veineuse ou artérielle (primitive ou secondaire), le diabète, la calcinose scrotale, les myosites ossifiantes, les ossifications ectopiques post-traumatiques et toute autre maladie ou condition pathologique par dépôt(s) de cristaux de calcium, en particulier d'hydroxyapatite ou de pyrophosphate de calcium.

Le thiosulfate de sodium administré par voie topique peut également permettre de traiter les conséquences d'une calcification ectopique.

Par « conséquences d'une calcification ectopique », on désigne toute complication liée à la présence d'une calcification ectopique. Cette complication peut par exemple être une complication de type fonctionnelle (en particulier une limitation des amplitudes articulaires), une douleur, une complication de type trophique (en particulier une ischémie ou une nécrose des tissus cutanés et/ou sous cutanés), ou une infection.

Afin de faciliter l'administration topique du thiosulfate de sodium et pour faciliter son accès au site de la calcification à traiter, le thiosulfate de sodium est formulé dans une composition pharmaceutique qui comprend en outre une émulsion hydrophile-dans-lipophile.

### Compositions pharmaceutiques comprenant le thiosulfate de sodium

Les compositions pharmaceutiques pour la mise en oeuvre du traitement selon l'invention contiennent du thiosulfate de sodium en quantité efficace pour atteindre le but recherché. En outre, les compositions pharmaceutiques pour la mise en oeuvre du traitement peuvent contenir des véhicules pharmaceutiquement acceptables appropriés comprenant des excipients et adjuvants qui facilitent la formulation du thiosulfate de sodium dans des préparations qui peuvent être utilisés pharmaceutiquement. L'expression "pharmaceutiquement acceptable" englobe tout véhicule qui n'interfère pas négativement avec l'efficacité de l'ingrédient actif sur les calcifications ou ossifications, et qui n'est pas toxique pour l'hôte auquel il est administré. En particulier, des véhicules pharmaceutiquement acceptables et appropriés pour une composition selon l'invention sont des véhicules convenant particulièrement à l'application de la composition sur la peau, un orifice, ou une muqueuse. Des véhicules pharmaceutiquement acceptables appropriés sont bien connus dans l'art et sont décrits par exemple dans Remington Pharmaceutical Sciences (Mack Publishing Company, Easton, USA, 1985), texte de référence standard dans ce domaine. Les véhicules pharmaceutiquement acceptables sont choisis en conformité avec le mode d'administration topique, la solubilité et la stabilité du thiosulfate de sodium.

La composition pharmaceutique comprenant le thiosulfate de sodium est formulée sous la forme d'une crème.

Les véhicules pharmaceutiquement acceptables utilisés dans les crèmes contiennent une émulsion hydrophile-dans-lipophile (H/L) comprenant une phase hydrophile dispersée et une phase lipophile continue.

Un autre aspect de la présente invention concerne donc une composition pharmaceutique comprenant du thiosulfate de sodium et une émulsion hydrophile-dans-lipophile. En particulier, la composition selon l'invention peut être une composition pharmaceutique comprenant du thiosulfate de sodium et une émulsion eau-dans-huile.

Une « émulsion » est un mélange, macroscopiquement homogène mais microscopiquement hétérogène, d'une phase lipophile et d'une phase hydrophile liquides non miscibles, comme l'eau et une huile. Une phase est dispersée dans la seconde phase sous forme de gouttelettes. Pour une « émulsion du type hydrophile-dans-lipophile », la phase hydrophile est dispersée dans la phase lipophile. La teneur en poids du thiosulfate de sodium dans la composition selon l'invention est de 5% à 25% par rapport au poids total de ladite composition. Préférentiellement encore, la teneur en poids du thiosulfate de sodium dans la composition selon l'invention est de 5% à 15%, en particulier de 8% à 12%, ou encore de 9,5% à 10,5% par rapport au poids total de ladite composition.

Les doses sont administrées selon les besoins individuels, l'effet désiré, la taille et la localisation des calcifications. Il est entendu que la dose administrée sera tributaire de l'âge, du sexe, de la santé et du poids du receveur, d'un traitement concomitant, le cas échéant, de la fréquence du traitement, et de la nature de l'effet désiré. La dose totale nécessaire pour chaque traitement peut être administrée par doses multiples ou en une seule dose. Une dose indicative pour la composition selon l'invention peut par exemple être d'environ 1 à 1,5 gramme à appliquer localement chaque jour, par exemple chaque soir et à laisser agir durant la nuit. L'émulsion hydrophile-dans-lipophile (H/L) selon l'invention comprend une composante hydrophile, une composante lipophile liquide à température ambiante, et une composante lipophile solide à température ambiante. Par « température ambiante », on entend une température comprise entre 18 °C et 25 °C.

La composante lipophile solide à température ambiante comprend de préférence une cire. Par « cire » on entend un composé solide ou substantiellement solide à température ambiante, et dont le point de fusion est généralement supérieur à 35 °C. Les cires peuvent être d'origine animale, végétale, minérale ou synthétique.

La composante lipophile solide à température ambiante peut par exemple comprendre de la cire d'abeille, telle que de la cire blanche ou de la cire jaune, du blanc de baleine, du palmitate de cétyle, de la cire de carnauba, de la cire de candelilla, de la cire d'Ouricoury, de la cire du Japon, de la cire de fibre de liège ou de canne à sucre, une cire de paraffine, de la vaseline, une cire de lignite, une cire microcristalline, de la lanoline cireuse, de l'ozokérite ou de la cérésine, une huile hydrogénée comme de l'huile de ricin hydrogénée, une cire de silicone, une cire végétale (ou un beurre), un alcool gras solide à température ambiante, une cire synthétique comme la cire de polyéthylène ou de Fischer Tropsch, ou un mélange de ceux-ci.

La teneur en poids de chacune des composantes comprises dans la composition selon l'invention peut varier. De préférence, la composante lipophile solide à température ambiante est présente avec une teneur en poids de 5% à 28%, de préférence de 8% à 25%, préférentiellement encore de 10% à 23%, par rapport au poids total de ladite composition.

Par ailleurs, le rapport en poids de la composante lipophile liquide à température ambiante sur la composante hydrophile liquide à température ambiante est de préférence de 0,8 à 1,9, de préférence de 1 à 1,9, de préférence de 1,1 à 1,8, et encore préférentiellement de 1,15 à 1,75. D'une manière préférentielle, le rapport en poids de la composante lipophile liquide à température ambiante sur la composante hydrophile liquide à température ambiante est de 1 à 1,9 lorsque la composition pharmaceutique est destinée à être utilisée chez un enfant. Elle peut varier de 0,8 à 1,9 lorsque la composition pharmaceutique est destinée à être utilisée chez un adulte.

On désigne par « composante lipophile liquide » une substance composée de molécules identiques ou différentes ayant des propriétés hydrophobes et liquide à température ambiante. La composante lipophile liquide peut en particulier comprendre une huile. Les huiles peuvent être d'origine végétale, animale, minérale ou synthétique.

La composante lipophile liquide à température ambiante peut par exemple comprendre une huile d'origine animale telle que l'huile de vison, l'huile de foie de morue ou de flétan, l'huile de tortue marine, une huile minérale telle que l'huile de paraffine, une huile synthétique comme le perhydrosqualène, les alcools gras, les amides grasses, les acides ou les esters gras, le Benzoate de 2-éthylphenyle, le palmitate d'octyle, le lanolate d'isopropyle, les triglycérides dont ceux des acides caprique/caprylique, le dicaprylyl carbonate, les esters et éthers gras oxyéthylénés ou oxypropylénés; les huiles siliconées (cyclométhicone, polydiméthysiloxanes ou PDMS) ou fluorées, les polyalkylènes, les trimellitates de trialkyle comme le trimellitate de tridécyle, une huile végétale, ou un mélange de ceux-ci. De manière préférentielle, la composante lipophile liquide à température ambiante selon l'invention comprend au moins une huile d'origine végétale.

Par « végétale » ou « d'origine végétale », on entend une substance extraite ou issue d'une ou plusieurs parties d'un ou plusieurs végétaux. Ceci exclut donc en particulier les composés issus d'un animal.

La ou les huiles végétales de l'invention sont de préférence choisies parmi le groupe consistant en : les huiles d'origine végétale ou un mélange d'huiles d'origine végétale, les triglycérides liquides d'acides gras comportant de 4 à 10 atomes de carbone comme les triglycérides des acides heptanoïque ou octanoïque d'origine végétale, ou les huiles de tournesol, de maïs, de soja, de courge, de sésame, de noisette, d'amande, d'abricot, d'amande douce, de macadamia, d'arara, de coriandre, de ricin, d'avocat, de rosier muscat, de fruits d'églantier, de pépins de raisin, de cassis, de jojoba, d'olive, d'arachide, de coco, de noyaux ; les huiles hémi-synthétiques telles que les triglycérides des acides caprylique/caprique d'origine végétale ; l'huile de jojoba, l'huile de beurre de karité ; les alcanes linéaires d'origine végétale, comprenant de préférence de 7 à 14 atomes de carbone, et l'un quelconque de leurs mélanges.

La « composante hydrophile liquide », en particulier la « solution aqueuse », peut comprendre de l'eau et éventuellement des solvants et/ou additifs hydrosolubles. Comme solvants hydrosolubles on peut citer notamment les alcools comportant de 2 à 8 atomes de carbone, notamment de 2 à 6 atomes de carbone comme l'éthanol. Comme polyols, on peut citer par exemple le glycérol, le butylène glycol et les polyéthylènes glycols. La phase aqueuse peut optionnellement contenir d'autres additifs comme des actifs hydrosolubles, des conservateurs, des sels, des gélifiants, des charges, des polymères hydrosolubles ou hydrodispersibles, des colorants hydrosolubles, etc.

Les « additifs » selon l'invention peuvent par exemple être des stabilisants, des excipients, des tampons, ou des conservateurs. Les solvants et/ou additifs hydrosolubles optionnellement présents dans la composante hydrophile liquide à température ambiante peuvent par exemple être choisis parmi des gommes, des agents tensioactifs anioniques, cationiques, amphotères ou non ioniques, des agents tensioactifs silicones, des gommes, des résines, des agents dispersants, des polymères semi-cristallins, des agents structurants hydrophiles, des agents antioxydants, des conservateurs, des parfums, des neutralisants, des agents antiseptiques, des filtres UV, des actifs cosmétiques, telles que des vitamines hydrophiles, des agents hydratants, émollients ou protecteurs de collagène, et leurs mélanges.

De préférence, l'invention concerne une composition pharmaceutique comprenant du thiosulfate de sodium et une émulsion hydrophile-dans-lipophile, dans laquelle l'émulsion comprend, en poids par rapport au poids total de l'émulsion : (i) 7-30%, et de préférence 10-25%, d'une composante lipophile solide à température ambiante; et (ii) 45-65%, et de préférence 50-60%, d'une composante lipophile liquide à température ambiante; et (iii) 15-40%, et de préférence 20-35%, d'une composante hydrophile liquide à température ambiante.

De préférence, la composante lipophile solide à température ambiante comprend une cire. De préférence, la composante lipophile liquide à température ambiante comprend une huile végétale. De préférence, la composante hydrophile liquide à température ambiante comprend au moins de l'eau, et optionnellement un ou des solvants et/ou additifs hydrosolubles, en particulier un parfum.

En particulier, l'émulsion hydrophile-dans-lipophile peut être le Cérat de Galien ou la Cold Cream.

Le « Cérat de Galien » est une émulsion notamment utilisée comme base pour la formulation de préparations officinales dermatologiques. La formule originale contient de la cire d'abeille (ou cire blanche), de l'huile d'amande douce, de l'eau de rose, et du borate de sodium. Un exemple de formule possible pour le Cérat de Galien est donné ci-dessous :

| | |
|---|---|
| Cire d'abeille blanche | 13 g |
| Huile d'amande douce | 53,5 g |
| Eau de rose | 33 g |
| Borate de Sodium | 0,5 g. |

La « Cold Cream » est une autre émulsion utilisée comme base pour la formulation de préparations officinales dermatologiques, dont la formule diffère de celle du Cérat de Galien par la présence de blanc de baleine (ou de palmitate de cétyle), de teinture de benjoin et d'essence de rose. Un exemple de formule possible pour la Cold Cream est donné ci-dessous :

| | |
|---|---|
| Cire d'abeille blanche | 8 g |
| Blanc de baleine ou palmitate de cétyle | 16 g |
| Huile d'amande douce | 55 g |
| Eau de rose | 16 g |
| Essence de rose | 0,5 g |
| Teinture de benjoin | 4 g |
| Borate de Sodium | 0,5 g. |

Ainsi, une composition pharmaceutique plus particulière selon l'invention est une composition dans laquelle la teneur en poids du thiosulfate de sodium est de 5% à 25%, en particulier de 8% à 15%, ou de 8% à 12%, ou encore de 9,5% à 10,5% par rapport au poids total de ladite composition, et dans laquelle l'émulsion hydrophile-dans-lipophile est le Cérat de Galien ou la Cold Cream.

### Procédé de préparation des compositions pharmaceutiques selon l'invention

Un dernier aspect de l'invention concerne un procédé de préparation d'une composition selon l'invention, comprenant une étape de dispersion du thiosulfate de sodium dans une émulsion hydrophile-dans-lipophile, le thiosulfate de sodium ayant optionnellement été préalablement dissous dans une solution hydrophile.

Le procédé de préparation d'une composition selon l'invention comprend de préférence une première étape de réduction du thiosulfate de sodium en une poudre fine et homogène, par exemple par broyage ou micronisation. La poudre ainsi obtenue pourra dans un second temps être dispersée dans un véhicule pharmaceutiquement acceptable qui est une émulsion hydrophile-dans-lipophile.

Selon un premier mode de réalisation, la poudre de thiosulfate de sodium peut être directement dispersée dans l'émulsion hydrophile-dans-lipophile, par exemple par mélange progressif de la poudre de thiosulfate de sodium et de l'émulsion hydrophile-dans-lipophile, jusqu'à obtention d'un produit macroscopiquement et/ou microscopiquement homogène.

Selon un second mode de réalisation, la poudre de thiosulfate de sodium obtenue lors de la première étape peut être dissoute dans une fraction de la composante hydrophile entrant dans la composition du véhicule pharmaceutiquement acceptable lors d'une deuxième étape, de sorte à obtenir une solution aqueuse contenant le thiosulfate de sodium. Une troisième étape consiste alors à mélanger cette solution aqueuse contenant le thiosulfate de sodium avec le reste du véhicule pharmaceutiquement acceptable, c'est-à-dire avec une émulsion comprenant la composante lipophile totale entrant dans la composition du véhicule pharmaceutiquement acceptable et la fraction de la composante hydrophile du véhicule pharmaceutiquement acceptable non utilisée dans l'étape de dissolution du thiosulfate de sodium.

En particulier, selon ce second mode de réalisation, le procédé de préparation d'une composition selon l'invention peut comprendre les étapes qui consistent à :
1) broyer ou microniser le thiosulfate de sodium jusqu'à obtention d'une poudre fine et homogène
2) faire fondre la composante lipophile solide du véhicule pharmaceutiquement acceptable dans la composante lipophile liquide du véhicule pharmaceutiquement acceptable, pour obtenir la composante lipophile totale
3) mélanger les ingrédients constituant la composante hydrophile liquide du véhicule pharmaceutiquement acceptable
4) mélanger ensemble la composante lipophile totale obtenue à l'étape 2) avec une première fraction de la composante hydrophile liquide obtenue à l'étape 3) jusqu'à obtention d'un mélange homogène
5) dissoudre la poudre de thiosulfate de sodium obtenue à l'étape 1 dans une deuxième fraction de la composante hydrophile liquide obtenue à l'étape 3), pour obtenir une solution aqueuse contenant le thiosulfate de sodium
6) ajouter la solution aqueuse contenant le thiosulfate de sodium obtenue à l'étape 5 au mélange obtenu à l'étape 4, et mélanger jusqu'à obtenir une composition pharmaceutique homogène.

Selon ce second mode de réalisation du procédé de préparation d'une composition selon l'invention, la composante hydrophile est séparée en deux fractions, lesdites « première et deuxième fractions ». Les étapes 4) et 5) peuvent être mises en oeuvre dans n'importe quel ordre. De préférence, l'étape 5) est réalisée juste avant l'étape 6), afin de limiter la dégradation du thiosulfate de sodium dans la solution aqueuse.

Alternativement, selon ce second mode de réalisation, le procédé de préparation d'une composition selon l'invention peut comprendre les étapes qui consistent à :
1) broyer ou microniser le thiosulfate de sodium jusqu'à obtention d'une poudre fine et homogène
2) faire fondre la composante lipophile solide du véhicule pharmaceutiquement acceptable dans la composante lipophile liquide du véhicule pharmaceutiquement acceptable, pour obtenir la composante lipophile totale
3) optionnellement, mélanger une partie des ingrédients constituant la composante hydrophile liquide du véhicule pharmaceutiquement acceptable
4) mélanger ensemble la composante lipophile totale obtenue à l'étape 2) avec le mélange hydrophile liquide obtenu à l'étape 3), ou avec un des ingrédients constituant la composante hydrophile liquide du véhicule pharmaceutiquement acceptable, jusqu'à obtention d'un mélange homogène
5) dissoudre la poudre de thiosulfate de sodium obtenue à l'étape 1 dans le(s) ingrédient(s) constituant la composante hydrophile liquide du véhicule pharmaceutiquement acceptable n'ayant pas été utilisé(s) dans l'étape 4), pour obtenir une solution aqueuse contenant le thiosulfate de sodium
6) ajouter la solution aqueuse contenant le thiosulfate de sodium obtenue à l'étape 5 au mélange obtenu à l'étape 4, et mélanger jusqu'à obtenir une composition pharmaceutique homogène.

L'homogénéité de la composition pharmaceutique obtenue peut par exemple être contrôlée par microscopie en évaluant la taille des globules hydrophiles dispersés dans la phase continue lipophile, ainsi que leur nombre dans un champ visuel d'une dimension donnée. Un tel contrôle peut également permettre de s'assurer de la bonne reproductibilité du procédé de préparation de la composition pharmaceutique selon l'invention. Par ailleurs, la teneur en ingrédient(s) actif(s) peut par exemple être contrôlée par chromatographie liquide haute performance. Enfin, certains paramètres physico-chimiques peuvent être contrôlés, par exemple la fourchette de pH dans laquelle la composition pharmaceutique selon l'invention se situe.

La composition pharmaceutique selon l'invention pourra de préférence être conditionnée en tube en aluminium vernis intérieurement pour produits médicamenteux à usage humain. Un tel conditionnement permet en effet de limiter le contact de la composition pharmaceutique avec l'oxygène de l'air (facteur de dégradation du thiosulfate de sodium) et la contamination microbienne lors de l'utilisation. Le tube sera ensuite préférentiellement conservé dans un endroit frais et sec, à l'abri des acides et des substances oxydantes.

### EXEMPLE 1 : Matériel et Méthode

Une série de formulations contenant des concentrations croissantes en thiosulfate de sodium (5, 10, 15, 20 et 25%) a été préparée (voir Tableau 1). Il a ensuite été vérifié que les compositions préparées répondaient aux critères de qualité suivants : pas de dégagement d'odeur soufrée, homogénéité visuelle et homogénéité à l'étalement.

**Tableau 1 :Composition des formulations de thiosulfate de sodium en émulsion hydrophile-dans-lipophile.**

| **Nom de la formulation** | **Ff5** | **Fi10** | **Ff10** | **Ff15** | **Ff20** | **Ff25** |
|---|---|---|---|---|---|---|
| Thiosulfate de sodium | 5 | 10 | 10 | 15 | 20 | 25 |
| Emulsion hydrophile/lipophile | 95 | 90 | 90 | 85 | 80 | 75 |
| Eau versable | 0 | 6,67 | 0 | 0 | 0 | 0 |
| Cire blanche | 12,35 | 10,8329 | 11,7 | 11,05 | 10,4 | 9,75 |
| Huile amande douce | 50,83 | 44,58 | 48,15 | 45,48 | 42,8 | 40,13 |
| Eau distillée rose | 31,35 | 27,50 | 29,70 | 28,05 | 26,4 | 24,75 |
| Borate de sodium | 0,48 | 0,42 | 0,45 | 0,43 | 0,4 | 0,38 |
| Composante lipophile / composante hydrophile | 63,18/36,83 | 55,41/44,59 | 59,85/40,15 | 56,53/43,48 | 53,2/46,8 | 49,88/50,13 |
| Composante hydrophile liquide / composante lipophile liquide | 36,83/50,83 | 44,59/44,58 | 40,15/48,15 | 43,48/45,48 | 46,8/42,8 | 50,13/40,13 |
| Rapport composante lipophile liquide / composante hydrophile liquide | 1,38 | 1,00 | 1,20 | 1,05 | 0.91 | 0,80 |

### EXEMPLE 2

Un garçon âgé de 12 ans souffrant d'un syndrome de calcinose tumorale familiale s'est présenté avec une importante calcification sous-cutanée au niveau de la face postérieure de son coude gauche. La taille de la calcification entrainait une réduction de la mobilisation du coude. Etant donné l'altération fonctionnelle et les limites potentielles d'un traitement chirurgical, un traitement percutané par le thiosulfate de sodium a été décidé. La préparation était constituée de thiosulfate de sodium dispersé dans une base lipophile (10/90, poids/poids) (formulation notée « Fi10 » dans le Tableau 1). L'enfant ne recevait aucune autre médication de type pharmacologique ou mécanique.

Le patient a appliqué localement environ 1 à 1,5 gramme du traitement chaque soir et l'a laissé agir durant la nuit. Aucun effet secondaire, ni général, ni local, n'a été rapporté ou observé. Après six mois de traitement, l'examen médical a confirmé une amélioration drastique, aucune lésion sous-cutanée n'étant visible et la mobilisation du coude étant redevenue normale. Une analyse par rayons X a confirmé l'amélioration.

Le thiosulfate de sodium est un agent prometteur pour le traitement des calcifications sous-cutanées. Des publications portant sur l'administration intraveineuse du thiosulfate de sodium ont rapporté des résultats prometteurs. Cependant, des problèmes de sécurité persistent concernant l'administration systémique du thiosulfate de sodium à cause d'effets secondaires digestifs, métaboliques et osseux. Par conséquent, une application locale du thiosulfate de sodium pourrait combiner l'efficacité de ce traitement avec des effets secondaires systémiques moindres ou inexistants. Deux publications rapportent des cas de traitements réussis de calcifications microscopiques dystrophiques avec du thiosulfate de sodium appliqué par voie cutanée, mais dans les deux articles le traitement était associé avec d'autres médications. A notre connaissance, il s'agit donc là du premier cas rapporté de disparition d'une calcification sous-cutanée métastatique de taille importante suite à une administration topique de thiosulfate de sodium, sans association à d'autres traitements. La taille initiale de la calcification, l'amélioration drastique, et l'évolution positive en une relativement courte période suggèrent que cette évolution ne pourrait pas être spontanée.

Ces données montrent que le thiosulfate de sodium peut être utilisé en administration topique pour permettre un traitement efficace et sûr, non seulement des calcifications sous-cutanées métastatiques, mais aussi de toutes les calcifications des tissus mous, quelle qu'en soit leur cause, indépendamment des mécanismes mis en jeu.

### EXEMPLE 3

Un enfant de 9 ans, 27 kg, souffrant d'hétéroplasie osseuse progressive présentait des ossifications importantes responsables d'une limitation fonctionnelle au niveau du pied droit : à la cheville, avec une surface estimée à 10 cm x 5 cm, et au creux poplité avec 2 plaques, de surface estimée à 6 cm x 4 cm chacune. Aucune amélioration n'avait été observée malgré un traitement par anti-inflammatoires non stéroïdiens et étidronate disodique. A la faveur d'une poussée, un traitement par une composition selon l'invention à base de thiosulfate de sodium dispersé à 10% dans une émulsion hydrophile/lipophile a été tenté (formulation notée « Ff10 » dans le Tableau 1). L'enfant ne recevait aucune autre médication de type pharmacologique ou mécanique.

Afin de juger de l'efficacité du traitement après 6 mois, il avait notamment été prévu des explorations par imagerie (TDM scanner) avant traitement puis à 6 mois, et des analyses biologiques recherchant en particulier une methémoglobinémie. Ces explorations ont été réalisées en complément du bilan habituel comprenant l'exploration de la fonction rénale et de la fonction hépatique, ainsi que le bilan phosphocalcique.

Après 3 mois de traitement, le clinicien notait déjà une peau plus mobile sur l'ossification.

Du point de vue de l'efficacité du traitement, les résultats à 6 mois ont confirmé la non adhérence de la peau à l'ossification sous-jacente. De plus, les extrusions cutanées de calcium avaient totalement disparu. Enfin, le scanner a révélé une taille stabilisée des ossifications, alors que le processus était évolutif avant l'application du traitement topique.

Du point de vue de la tolérance, aucun changement de la fonction rénale et de la fonction hépatique n'ont été observés, et la methémoglobinémie était inférieure à 1%.

Ces résultats ont conduit à poursuivre le traitement, avec une concentration plus forte, de 15% (formulation notée « Ff15 » dans le Tableau 1).

### EXEMPLE 4

Le troisième cas concerne une patiente de 72 ans souffrant d'une sclérodermie de type CREST diagnostiquée depuis plus de 15 ans. Depuis longtemps, elle présentait des calcifications digitales compliquées de temps à autres d'ulcérations et, plus récemment, des calcifications confluentes sur la face externe des deux avant-bras. Le traitement général habituel de cette patiente associait prednisone à 5 mg et bosentan. Elle ne bénéficiait d'aucun traitement local.

Avec l'extension du phénomène de calcifications (apparition sur les avant-bras) et l'existence d'une symétrie, l'administration topique d'une formulation selon l'invention a été décidée, à la concentration de 10% sur un bras (formulation notée « Ff10 » dans le Tableau 1) et de 25% sur l'autre bras (formulation notée « Ff25 » dans le Tableau 1).

Après 3 mois de traitement, les résultats montrent une tolérance parfaite aux deux concentrations. Une « légère amélioration » a été notée par la patiente, avec une intensité relativement supérieure de la concentration à 25%.

Le même type d'évaluations que celles énoncées dans l'Exemple 2 est mis en place après 6 mois, adapté à la sclérodermie.

### EXEMPLE 5

Des solutions des sels de calcium suivants ont été préparées par mélange d'une partie de poudre calcique dans 30 parties d'eau stérile versable : gluconate, chlorure, carbonate, et phosphates monocalcique, dicalcique et tricalcique. Des comportements attendus pour ces solutions ont été vérifiés, notamment une hydrosolubilité croissante en passant de carbonate à chlorure, en passant par les autres sels.

Une partie de chaque formulation décrite dans l'Exemple 1 est ajoutée à 10 parties de chacune des solutions calciques (PA x% / sel calcium) précédemment préparées. La durée de contact avec la composition pharmaceutique selon l'invention étant d'environ 7 ou 8h, les simulations *in vitro* sont estimées à 4h pour une agitation à 60 rpm, à 2h pour 120 rpm etc. L'ensemble est mélangé à une vitesse de 120 rpm, pendant 2h avec un agitateur Rayneri.

L'influence des formulations sur la dissolution des sels calciques est quantifiée par des techniques néphélémétriques et turbidimétriques. La turbidité est d'autant plus faible que la formulation est efficace en matière de dissolution du sel de calcium. A l'inverse, elle est importante si la préparation n'a pas d'influence sur le sel de calcium étudié.

Certains mélanges ne répondant pas de façon satisfaisante à ces méthodes, une spectrophotométrie dans le visible est réalisée. Afin d'obtenir le maximum de sensibilité, la longueur d'onde d'un pic d'absorption est recherchée par balayage avant la réalisation des mesures d'absorbance. Des dilutions sont effectuées autant que nécessaire.

Des solutions témoins accompagnent les mesures.

## Revendications

1. Composition pharmaceutique comprenant du thiosulfate de sodium et une émulsion hydrophile-dans-lipophile, dans laquelle la teneur en poids du thiosulfate de sodium est de 5% à 25% par rapport au poids total de ladite composition, et dans laquelle l'émulsion hydrophile-dans-lipophile comprend une composante lipophile solide à température ambiante, une composante lipophile liquide à température ambiante et une composante hydrophile liquide à température ambiante.

2. Composition pharmaceutique selon la revendication 1, dans laquelle la composante lipophile solide à température ambiante est présente avec une teneur en poids de 5% à 28% par rapport au poids total de ladite composition.

3. Composition pharmaceutique selon la revendication 1 ou 2, dans laquelle le rapport en poids de la composante lipophile liquide à température ambiante sur la composante hydrophile liquide à température ambiante est de 0,8 à 1,9.

4. Composition pharmaceutique selon l'une quelconque des revendications 1 à 3, dans laquelle le rapport en poids de la composante lipophile liquide à température ambiante sur la composante hydrophile liquide à température ambiante est de 1 à 1,9.

5. Composition pharmaceutique selon l'une quelconque des revendications 1 à 4, dans laquelle l'émulsion comprend en poids par rapport au poids total de l'émulsion :
a) 7-30% d'une composante lipophile solide à température ambiante; et
b) 45-65% d'une composante lipophile liquide à température ambiante; et
c) 15-40% d'une composante hydrophile liquide à température ambiante.

6. Composition pharmaceutique selon l'une quelconque des revendications 1 à 5, dans laquelle ladite composante lipophile liquide à température ambiante comprend au moins une huile végétale.

7. Composition pharmaceutique selon l'une quelconque des revendications 1 à 6, dans laquelle ladite composante hydrophile liquide à température ambiante comprend de l'eau, et optionnellement un ou des solvants et/ou additifs hydrosolubles.

8. Composition pharmaceutique selon l'une quelconque des revendications 1 à 7, dans laquelle la teneur en poids du thiosulfate de sodium est de 8% à 12% par rapport au poids total de ladite composition, et dans laquelle l'émulsion hydrophile-dans-lipophile est le Cérat de Galien.

9. Composition pharmaceutique selon l'une quelconque des revendications 1 à 8 pour son utilisation pour une administration topique pour le traitement d'une calcification ectopique et/ou de ses conséquences chez un individu, le thiosulfate de sodium étant sous la forme d'une composition pharmaceutique comprenant en outre une émulsion hydrophile-dans-lipophile.

10. Composition pharmaceutique pour son utilisation selon la revendication 9, la calcification étant une calcification cutanée ou sous-cutanée.

11. Composition pharmaceutique pour son utilisation selon la revendication 9 ou 10, la calcification étant une calcification métastatique, dystrophique, iatrogène, ou idiopathique, une calcification associée à de la calciphylaxie, ou une ossification ectopique sous-cutanée.

12. Composition pharmaceutique pour son utilisation selon l'une quelconque des revendications 9 à 11, la calcification étant associée à une maladie ou condition pathologique sélectionnée dans le groupe constitué de l'hyperparathyroidie primaire, l'intoxication à la vitamine D, le syndrome des buveurs de lait, l'hypercalcémie, l'hyperparathyroidie secondaire, l'insuffisance rénale, les hyperphosphatémies, en particulier génétiques, la sclérodermie, la dermatomyosite, en particulier la forme juvénile, les connectivités mixtes, le lupus, le syndrome CREST, le syndrome d'Elhers-Danlos, le pseudo xanthome élastique, le syndrome de Werner, les porphyries cutanées tardives, la pseudo hypoparathyroidie, la pseudo-pseudo hypoparathyroidie, l'insuffisance veineuse ou artérielle (primitive ou secondaire), le diabète, la calcinose scrotale, les myosites ossifiantes, les ossifications ectopiques post-traumatiques et toute autre maladie ou condition pathologique par dépôt(s) de cristaux de calcium, en particulier d'hydroxyapatite ou de pyrophosphate de calcium.

13. Procédé de préparation d'une composition selon l'une quelconque des revendications 1 à 8, comprenant une étape de dispersion du thiosulfate de sodium dans une émulsion hydrophile-dans-lipophile, le thiosulfate de sodium ayant optionnellement été préalablement dissous dans une solution hydrophile.

## Patentansprüche

1. Pharmazeutische Zusammensetzung, umfassend Natriumthiosulfat und eine Hydrophil-in-lipophil-Emulsion, wobei der Gewichtsanteil des Natriumthiosulfats 5 % bis 25 % im Verhältnis zum Gesamtgewicht der besagte Zusammensetzung beträgt, und wobei die Hydrophil-in-lipophil-Emulsion einen bei Raumtemperatur festen lipophilen Bestandteil, einen bei Raumtemperatur flüssigen lipophilen Bestandteil und einen bei Raumtemperatur flüssigen hydrophilen Bestandteil umfasst.

2. Pharmazeutische Zusammensetzung nach Anspruch 1, wobei der bei Raumtemperatur feste lipophile Bestandteil in einem Gewichtsanteil von 5 % bis 28 % im Verhältnis zum Gesamtgewicht der besagte Zusammensetzung vorhanden ist.

3. Pharmazeutische Zusammensetzung nach Anspruch 1 oder 2, wobei das Gewichtsverhältnis des bei Raumtemperatur flüssigen lipophilen Bestandteils zum bei Raumtemperatur flüssigen hydrophilen Bestandteil 0,8 bis 1,9 beträgt.

4. Pharmazeutische Zusammensetzung nach einem der Ansprüche 1 bis 3, wobei das Gewichtsverhältnis des bei Raumtemperatur flüssigen lipophilen Bestandteils zum bei Raumtemperatur flüssigen hydrophilen Bestandteil 1 bis 1,9 beträgt.

5. Pharmazeutische Zusammensetzung nach einem der Ansprüche 1 bis 4, wobei die Emulsion gewichtsmäßig im Verhältnis zum Gesamtgewicht der Emulsion umfasst:
a) 7-30 % eines bei Raumtemperatur festen lipophilen Bestandteils und
b) 45-65 % eines bei Raumtemperatur flüssigen lipophilen Bestandteils und
c) 15-40 % eines bei Raumtemperatur flüssigen hydrophilen Bestandteils.

6. Pharmazeutische Zusammensetzung nach einem der Ansprüche 1 bis 5, wobei der besagte bei Raumtemperatur flüssige lipophile Bestandteil mindestens ein Pflanzenöl umfasst.

7. Pharmazeutische Zusammensetzung nach einem der Ansprüche 1 bis 6, wobei der besagte bei Raumtemperatur flüssige hydrophile Bestandteil Wasser und optional ein oder Lösungsmittel und/oder wasserlösliche Zusatzstoffe umfasst.

8. Pharmazeutische Zusammensetzung nach einem der Ansprüche 1 bis 7, wobei der Gewichtsanteil des Natriumthiosulfats 8 % bis 12 % im Verhältnis zum Gesamtgewicht der besagte Zusammensetzung beträgt und wobei die Hydrophil-in-lipophil-Emulsion Cold Cream ist.

9. Pharmazeutische Zusammensetzung nach einem der Ansprüche 1 bis 8 für ihre topische Verabreichung zur Behandlung einer ektopischen Kalzifizierung und/oder ihrer Folgen bei einer Person, wobei das Natriumthiosulfat in Form einer pharmazeutischen Zusammensetzung vorliegt, die ferner eine Hydrophil-in-lipophil-Emulsion umfasst.

10. Pharmazeutische Zusammensetzung für ihre Verwendung nach Anspruch 9 oder 10, wobei die Kalzifizierung eine kutane oder subkutane Kalzifizierung ist.

11. Pharmazeutische Zusammensetzung für ihre Verwendung nach Anspruch 9 oder 10, wobei die Kalzifizierung eine metastatische, dystrophische, iatrogene oder idiopathische Kalzifizierung, eine Kalzifizierung in Kombination mit der Kalziphylaxie oder eine subkutane ektopische Ossifikation ist.

12. Pharmazeutische Zusammensetzung für ihre Verwendung nach einem der Ansprüche 9 bis 11, wobei die Kalzifizierung mit einer Erkrankung oder pathologischen Bedingung kombiniert ist, die aus der Gruppe ausgewählt ist, die vom primären Hyperparathyreoidismus, von der Vitamin-D-Intoxikation, dem Milchtrinkersyndrom, der Hyperkalzämie, dem sekundären Hyperparathyreoidismus, der Niereninsuffizienz, den vor allem genetischen Hyperphosphatämien, der Sklerodermie, der vor allem juvenilen Dermatomyositis, den Mischsymptomen, dem Lupus, dem CREST-Syndrom, dem Elhers-Danlos-Syndrom, dem Pseudoxanthoma elasticum, dem Werner-Syndrom, den tardiven kutanen Porphyrieen, dem Pseudo-Hypoparathyreoidismus, dem Pseudo-Pseudo Hypoparathyreoidismus, der venösen oder arteriellen Insuffizienz (primitiv oder sekundär), dem Diabetes, der skrotalen Kalzinose, den ossifizierenden Myositiden, den posttraumatischen ektopischen Ossifikationen und jeder anderen Erkrankung oder pathologischen Bedingung durch Ablagerung(en) von Kalziumkristallen, insbesondere von Kalziumhydroxyapatit oder -pyrophosphat, gebildet ist.

13. Herstellungsverfahren einer Zusammensetzung nach einem der Ansprüche 1 bis 8, umfassend einen Dispersionsschritt des Natriumthiosulfats in einer Hydrophil-in-lipophil-Emulsion, wobei das Natriumthiosulfat zuvor optional in einer hydrophilen Lösung aufgelöst wurde.

## Claims

1. A pharmaceutical composition comprising sodium thiosulfate and a hydrophile-in-lipophile emulsion, wherein the content by weight of sodium thiosulfate is from 5% to 25% relative to the total weight of said composition, and wherein the hydrophile-in-lipophile emulsion comprises a lipophilic component that is solid at room temperature, a lipophilic component that is liquid at room temperature, and a hydrophilic component that is liquid at room temperature.

2. A pharmaceutical composition according to claim 1, wherein the lipophilic component that is solid at room temperature is present at a content by weight of 5% to 28% relative to the total weight of said composition.

3. A pharmaceutical composition according to claim 1 or 2, wherein the ratio by weight of the lipophilic component that is liquid at room temperature to that of the hydrophilic component that is liquid at room temperature is from 0.8 to 1.9.

4. A pharmaceutical composition according to any one of claims 1 to 3, wherein the ratio by weight of the lipophilic component that is liquid at room temperature to that of the hydrophilic component that is liquid at room temperature is from 1 to 1.9.

5. A pharmaceutical composition according to any one of claims 1 to 4, wherein the emulsion comprises, by weight relative to the total weight of the emulsion:
a) 7% - 30% of a lipophilic component that is solid at room temperature; and
b) 45% - 65% of a lipophilic component that is liquid at room temperature; and
c) 15% - 40% of a hydrophilic component that is liquid at room temperature.

6. A pharmaceutical composition according to any one of claims 1 to 5, wherein said lipophilic component that is liquid at room temperature comprises at least one vegetable oil.

7. A pharmaceutical composition according to any one of claims 1 to 6, wherein said hydrophilic component that is liquid at room temperature comprises water, and optionally one or more solvents and/or water soluble additives.

8. A pharmaceutical composition according to any one of claims 1 to 7, wherein the content by weight of sodium thiosulfate is from 8% to 12% relative to the total weight of said composition, and wherein the hydrophile-in-lipophile emulsion is Galen's Wax.

9. A pharmaceutical composition according to any one of claims 1 to 8 for use for topical administration for the treatment of an ectopic calcification and/or of the consequences thereof in an individual, the sodium thiosulfate being in the form of a pharmaceutical composition comprising, in addition, a hydrophile-in-lipophile emulsion.

10. A pharmaceutical composition for the use according to claim 9, the calcification being a cutaneous or subcutaneous calcification.

11. A pharmaceutical composition for the use according to claim 9 or 10, the calcification being a metastatic calcification, a dystrophic calcification, a iatrogenic calcification, an idiopathic calcification, a calcification associated with calciphylaxis, or a subcutaneous ectopic ossification.

12. A pharmaceutical composition for the use according to any one of claims 9 to 11, the calcification being associated with a disease or pathological condition selected from the group consisting of primary hyperparathyroidism, vitamin D intoxication, milk drinker's syndrome, hypercalcemia, secondary hyperparathyroidism, renal failure, hyperphosphatemia, in particular genetic hyperphosphatemia, scleroderma, dermatomyositis, in particular the juvenile form of dermatomyositis, mixed connective tissue diseases, lupus, CREST syndrome, Elhers-Danlos syndrome, pseudo xanthoma elasticum, Werner's syndrome, late cutaneous porphyria, pseudo hypoparathyroidism, pseudo pseudo-hypoparathyroidism, (primary or secondary) venous or arterial insufficiency, diabetes, scrotal calcinosis, ossifying myositis, post-traumatic ectopic ossifications and any other disease or pathological condition caused by calcium crystal deposit(s), in particular by hydroxyapatite or calcium pyrophosphate crystal deposit(s).

13. A method for preparing a composition according to any one of claims 1 to 8, comprising a step of dispersion of the sodium thiosulfate in a hydrophile-in-lipophile emulsion, the sodium thiosulfate having optionally been previously dissolved in a hydrophilic solution.
